# EUROPÄISCHE PATENTSCHRIFT

(11) **EP 1 108 782 B1**
(45) Veröffentlichungstag und Bekanntmachung des Hinweises auf die Patenterteilung: **19.10.2005**
(21) Anmeldenummer: 99125291.7
(22) Anmeldetag: 18.12.1999
(51) Int. Cl.: C12N 15/10, C07H 21/00

(54) **Verfahren und Zusammensetzungen zur Isolierung und Reinigung von Nukleinsäuren**
Methods and kits for isolating and purifying nucleic acids
Méthodes d'extraction et trousses de purification des acides nucléiques

(43) Veröffentlichungstag der Anmeldung: 20.06.2001
(73) Patentinhaber: Gen-IAL GEN - Institut für Angewandte, Laboranalysen GmbH, 53840 Troisdorf (DE)
(72) Erfinder: Schönling, Jutta, Dr., 50823 Köln (DE); Mücher, Gabriele, Dr., 53859 Niederkassel (DE)
(74) Vertreter: Godemeyer, Thomas

(56) Entgegenhaltungen:
- MILLER S.A. ET AL.: "A simple salting out procedure for extracting DNA from human nucleated cells" NUCLEIC ACID RESEARCH, Bd. 16, Nr. 3, 1988, Seite 1215 XP000906918 OXFORD, GB
- TURTINEN L.W., DURAN B.D.: "Protein salting-out method applied to genomic DNA isolation from fish whole blood" BIOTECHNIQUES, Bd. 24, Nr. 2, 1998, Seiten 238-239, XP000906908
- LAITINEN J. ET AL.: "A nontoxic and versatile protein salting-out method for isolation of DNA" BIOTECHNIQUES, Bd. 17, Nr. 2, 1994, Seiten 316--322, XP000906961
- HURLEY C.K. ET AL.: "HLA Class I and II DNA-based typing. Sequence specific oligonucleotide probe typing. Technical manual / Reference protocols. Version 1.1" THIRTEENTH INTERNATIONAL HISTOCOMPATIBILITY WORKSHOP , [Online] 4. November 1998 (1998-11-04), XP002137318 Retrieved from the Internet: <URL:http://www.ihwc.org/protocols/man1.ht m> [retrieved on 2000-05-04]
- BOWTELL D.: "DNA Extraction. Mouse and Human Genomic DNA Extraction." SIGNAL TRANSDUCTION LABORATORY MANUAL, [Online] [CD-ROM] 23. November 1998 (1998-11-23), XP002137319 Retrieved from the Internet: <URL:http://www.pmci.unimelb.edu.au/manual /molbiol/mb030.htm> [retrieved on 2000-05-11]

## Beschreibung

Gegenstand der Erfindung ist ein Verfahren zur Isolierung und Reinigung von Nukleinsäuren, insbesondere von genomischer DNA aus einem organischen Material. Weiterhin betrifft die Erfindung einen Kit zur Verwendung in dem Verfahren.

Im Zuge der Verbreitung gentechnischer Verfahren zur Beeinflussung von Eigenschaften von Mikroorganismen, Pflanzen und damit auch Nahrungsmitteln wurden qualitative und quantitative Nachweise gentechnisch veränderter Organismen und pflanzlicher Zusätze, beispielsweise Soja, Mais oder Weizen, entwickelt. Für diese Nachweisverfahren müssen die Nukleinsäuren aus den entsprechenden Substraten gewonnen werden. Dazu muß die DNA von guter Qualität, sowie hochmolekular sein. Weiterhin muß die isolierte DNA in einer Sauberkeit vorliegen, die für molekulargenetische Arbeiten und Nachweisverfahren wie PCR (polymerase chain reaction; Polymerase-Kettenreaktion) oder Spaltung der isolierten DNA mit Restriktionsenzymen ausreicht.

Es sind bereits eine Reihe von DNA-lsolationsmethoden beschrieben worden, die in erster Linie zur Isolierung von DNA aus einem ganz bestimmten Substrat, beispielsweise grampositive oder gramnegative Bakterien, Pflanzen, Blut und Lebensmittel entwickelt wurden. Bei diesen Verfahren werden oft gesundheitsgefährdende Substanzen verwendet, wie Phenol, Cetyltrimethylammoniumbromid (CTAB) oder Guanidin-Hydrochlorid. Andere Verfahren nutzen teure Kunststoffartikel in Form von Säulchen oder Zentrifugenfilterröhrchen, die DNA-bindende Trägersubstanzen enthalten, wobei nicht recycelfähiger Plastikabfall anfällt.

So wird beispielsweise im Standardwerk Ausubel et al. (Current Protocols in Molekular Biology, Unit 2.3 und 2.4, John Wiley & Sons, Inc., 1994) die Isolierung von genomischer DNA aus Bakterien oder aus Pflanzengewebe beschrieben. Bei der Isolierung von genomischer DNA aus Bakterien werden die Zellen zunächst zur Lyse in einem Puffer aufgenommen. Nach dem Aufbrechen der Zellen wird Cetyltrimethylammoniumbromid (CTAB) hinzugegeben, das zur Entfernung von Proteinen und Polysacchariden dient. Bei der anschließenden Chloroform/ Isoamylalkohol-Extraktion und Zentrifugation werden die CTAB-Protein/Polysaccharid-Komplexe abgetrennt. Nach einer weiteren Extraktion mit Phenol/Chloroform/Isoamylalkohol wird die DNA mit Isopropanol gefällt, mit 70 %igem Ethanol gewaschen und in einem wäßrigen Puffer aufgenommen.

Bei der Isolierung von genomischer DNA aus Pflanzengewebe wird in Ausubel et al. entweder ein Verfahren einschließlich der CTAB-Behandlung oder die Isolierung mittels einer Cäsiumchlorid-(CsCl)-Gradientenzentrifugation vorgeschlagen. Bei dem letzten Verfahren wird zusätzlich Ethidiumbromid zur Detektion der DNA verwendet.

Gemäß der amtlichen Sammlung von Untersuchungsverfahren nach § 35 Lebensmittelbedarf-Gesetz umfaßt ein Schritt der Isolation von genomischer DNA aus Wurstwaren zum Nachweis von gentechnischen Veränderungen bestimmter Mikroorganismen eine Phenolextraktion.

In anderen Isolationsverfahren werden teure Kunststoffartikel in Form von Einweg-Säulchen enthaltend DNA-bindende Substanzen eingesetzt. So beschreibt beispielsweise die EP 0 616 639 ein Verfahren zur Isolierung und Reinigung von Nukleinsäuren, wobei nach Aufschluß der Zellen die Nukleinsäuren in den Säulchen zunächst an den Anionenaustauscher binden und die Nukleinsäuren durch Waschschritte gereinigt werden. Danach werden die Nukleinsäuren vom Anionenaustauscher abgelöst, um vom nachgeschalteten mineralischen Trägermaterial gebunden zu werden. Es folgen weitere Waschschritte mit wäßrigen alkoholischen Lösungen und schließlich die Elution in eine wäßrige Lösung.

Die Firma Roche bietet beispielsweise einen High Pure™ PCR Template Preparation Kit für die Isolierung und Aufreinigung von DNA, die für verschiedene molekularbiologische Methoden wie Restriktionsanalyse und PCR geeignet ist. Der Kit stellt unter anderem Zentrifugenfilterröhrchen bereit, enthaltend ein spezielles Glasfaservlies. Nach der Lyse der Bakterien, aus denen die DNA aufgereinigt werden soll, wird die Probe auf die Röhrchen gegeben und durch Zentrifugation im Glasfaservlies verteilt. Die DNA bindet an das Glasfaservlies und wird mittels verschiedener Lösungen gereinigt. Durch Zentrifugation werden die Waschlösungen und die abzutrennenden unerwünschten Zellreste entfernt. Danach wird die DNA durch Elution in einen Puffer vom Glasfaservlies abgelöst.

Hurley C.K. et al. (HLA class I und II DNA-based typing. Sequence specific oligonucleotide probe typing. Technical manual; Reference Protocols, Version 1.1, "Thirteenth International Histocompatibility Workshop) beschreibt im Abschnitt 1.4.1 einen Kit und ein Verfahren zur Isolierung von DNA aus Blut. Dabei beträgt die Salzkonzentration maximal ca. 1,5 M. Die SDS-Konzentration beträgt, nach Zugabe des Nuclear-Lysis-Puffers, der 10%igen SDS-Lösung, weiterem Nuclear-Lysis Puffer und der Proteinase_Lösung nicht mehr als 1%. Die SDS-Konzentration beträgt nach Zugabe der konzentrierten Salzkonzentration ca. 0,74 Gew-%. Das Verfahren eignet sich nicht dazu genomische DNA aus anderen organischen Materialien z.B. aus pflanzlichem Material zu gewinnen.

Martinez G. et al. ("Protein Salting-out Method Applied to Genomic DNA Isolation from Fish Whole Blood", Biotechniques, Vol. 24, Nr. 2 (1998) Seiten 238-239) beschreibt ein Verfahren zur Isolierung von DNA aus Blut. Hierbei wird ein Aussalzschritt zur Entfernung von Proteinen durchgeführt, wobei NaCl in einer Konzentration von 1,5 M verwendet wird. SDS wird in einer Konzentration von 0,5 Gew.% in der Lösung verwendet. Auch dieses Verfahren eignet sich nicht dazu genomische DNA aus anderen organischen Materialien z.B. aus pflanzlichem Material zu gewinnen.

Im oben genannten Stand der Technik wurden gleichzeitig hohe Salz- und SDS-Konzentrationen vermieden, um Salz-SDS-Präzipitate zu vermeiden, die vom Fachmann als störend für die DNA-Aufreinigung angesehen wurden. Aufgrund der geringen SDS-und Salz-Konzentrationen eignen sich die von Hurley C.K. et al. und Martinez G. et al. beschriebenen Verfahren aber nicht zur Gewinnung von genomischer DNA aus anderen organischen Materialien z.B. aus pflanzlichem Material, weil das Material nicht richtig aufgeschlossen werden kann, und neben Proteinen große Mengen an Polysacchariden nicht entfernt werden können.

Die Nachteile der Verfahren des Standes der Technik sind, daß gesundheitsgefährdende Substanzen verwendet werden, wie die organischen Lösungsmittel Phenol oder Cetyltrimethylammoniumbromid (CTAB), die getrennt entsorgt werden müssen. Bei anderen Verfahren werden beispielsweise teure DNA-bindende Einweg-Säulchen verwendet, wobei nicht recycelfähiger Plastikabfall anfällt. Weiterhin ist nachteilig, daß jedes Verfahren nur einen sehr begrenzten Einsatzbereich bezüglich der DNA-Quelle besitzt.

Die technische Aufgabe war es daher, ein Verfahren zur Isolierung und Reinigung von Nukleinsäuren zu entwickeln, wobei auf die Verwendung von umweltgefährdenden Bestandteilen, wie Phenol oder Cetyltrimethylammoniumbromid verzichtet wird, sowie kein nichtrecycelfähiger Plastikabfall anfällt. Weiterhin soll das Verfahren kostengünstig und grundsätzlich geeignet sein, um DNA aus verschiedensten organischen Materialien zu isolieren.

Die technische Aufgabe wird gelöst durch ein Verfahren zur Isolierung und Reinigung von Nukleinsäuren, insbesondere von genomischer DNA aus einem organischen Material umfassend die Schritte:
a) Aufnehmen des Nukleinsäuren-enthaltenden organischen Materials in einer Lösung A enthaltend mindestens 0,2 M NaCl oder KCl,
b) Zugeben von SDS (Natriumdodecylsulfat)
c) Zugeben einer Protease
d) Inkubation für 5 bis 120 min bei 50 bis 75 °C
e) Zugeben einer Lösung D enthaltend ein Salz ausgewählt aus der Gruppe NaCl, KCl, Natriumacetat, wobei eine Endkonzentration des Salzes von mindestens 2,3 M erreicht wird.
f) Abtrennen des unlöslichen Materials
g) Fällen der Nukleinsäuren mit einem Alkanol.

Bei dem erfindungsgemäßen Verfahren wird im Gegensatz zum Stand der Technik (z.B. Hurley C.K. et al. und Martinez G. et al.), eine weit höhere SalzKonzentration von mindestens 2,3 M verwendet. Diese Maßnahme erlaubt die sichere Entfernung von Proteinen und Polysacchariden, aber auch von großen Mengen von SDS, das zunächst für den Aufschluss von Pflanzenmaterial benötigt wird. Im Stand der Technik dagegen ist auch die verwendete SDS-Konzentration geringer.

Es wurde überraschenderweise festgestellt, daß mit diesem Verfahren Nukleinsäuren aus ganz verschiedenen organischen Materialien bzw. Organismen isoliert werden können. So ist dieses Verfahren geeignet zur Isolierung von Nukleinsäuren aus Hefen, Schimmelpilzen, aus grampositiven und gramnegativen Bakterien, phototrophe S-Purpur Bakterien (gramnegativ), Zellkulturen, Pflanzen, tierischen und menschlichen Geweben, Blut, Haarwurzeln, Speichel, sowie aus Fleisch, Fleischprodukten und anderen Lebensmitteln unterschiedlichster Verarbeitung und Zusammensetzung. Die Vorteile dieses Verfahrens sind weiterhin, daß die Verwendung von umweltgefährdenden Bestandteilen, wie Phenol oder CTAB (Cetyltrimethylammoniumbromid) vermieden wird und auch kein nichtrecycelfähiger Plastikabfall, beispielsweise in Form von DNA-bindenden Säulchen, anfällt. Das Verfahren erlaubt zudem die Isolation genomischer DNA in kurzer Zeit ohne Homogenisationsschritte. Die Qualität der gewonnen Nukleinsäuren ist hervorragend. Die DNA ist hochmolekular und wird in einer Reinheit erhalten, die für molekulargenetische Arbeiten wie PCR (polymerase chain reaction, Polymerase-Kettenreaktion) oder Spaltung mit Restriktionsenzymen ausreicht. Weiterhin sind die Ausbeuten, verglichen mit anderen Methoden, die DNA-bindende Säulchen einsetzen, sehr hoch. Zudem ist das Verfahren im Vergleich zu anderen Verfahren kostengünstiger.

In einem weiteren Verfahren erfolgen nach den Schritten a) und/oder b) zusätzlich Inkubationen bei 50 bis 75 °C. In einem bevorzugten Verfahren werden das Natriumdodecylsulfat und/oder eine Protease gemeinsam mit der Lösung A zugegeben. Mit dieser Vorgehensweise läßt sich das Verfahren weiter beschleunigen.

In einem weiteren bevorzugten Verfahren wird als Protease Proteinase K verwendet. Weiterhin wird bevorzugt zusätzlich eine RNAse zu dem Ansatz zugegeben. Insbesondere bei DNA-Präparationen aus Pflanzen und Fleisch könnte der Abbau von RNA erforderlich sein. Weiterhin wird vorzugsweise nach Schritt d) unlösliches Material abgetrennt. Der Ansatz weist nach Zugabe der Lösung D erfindungsgemäß eine Endkonzentration des Salzes von mindestens 2,3 M auf. Diese hohe Salzkonzentration erlaubt ein Ausfällen und Abtrennen von Polysacchariden und Proteinen.

In einem weiteren besonders bevorzugten Verfahren, erfolgt die Inkubation in Schritt d) für 15 bis 60 min bei 65 °C. Diese Auswahl der Parameter erlaubt eine hohe Ausbeute an DNA durch das erfindungsgemäße Verfahren.

In einem weiteren besonders bevorzugten Verfahren wird als Lösung A eine Lösung verwendet, die EDTA (Ethylendiamintetraessigsäure, Dinatriumsalz) und 0,4 bis 1 M NaCl enthält, wobei der pH-Wert der Lösung auf 7,5 bis 8,5 eingestellt ist.

In einem weiteren besonders bevorzugten Verfahren beträgt die Endkonzentration nach Zugabe des Natriumdodecylsulfats 1,0 bis 2,5 Gew.-%.

Weiterhin wird als Lösung D bevorzugt eine 2,5 bis 6 M NaCl-Lösung verwendet. Als Alkanol werden bevorzugt Ethanol oder Isopropanol verwendet.

In einem besonders bevorzugten Verfahren werden die Nukleinsäuren nach dem Fällen mit einer 70 bis 85 %igen Ethanol- oder Isopropanol-Lösung behandelt. Mit diesem Schritt werden die Salze von den Nukleinsäuren entfernt.

In einem weiteren besonders bevorzugten Verfahren wird das organische Material ausgewählt aus der prokaryotische Zellen, eukaryotische Zellen, Pflanzensamen, Pflanzenteile, Pflanzenöle, Nahrungsmittel, menschliche und tierische Gewebe, menschliche Ausscheidungen, Sperma, Speichel. Vorteilhafterweise hat sich nämlich gezeigt, daß das erfindungsgemäße Verfahren universell für ein großes Spektrum von Substraten zur Isolierung von Nukleinsäuren eingesetzt werden kann. Es können daher ohne Änderungen des Protokolls Nukleinsäuren aus verschiedenen Substraten auch automatisiert zur gleichen Zeit isoliert werden.

Weiterhin wird die technische Aufgabe durch Bereitstellung eines Kits zur Verwendung in dem erfindungsgemäßen Verfahren zur Isolierung und Reinigung von Nukleinsäuren gelöst, wobei der Kit enthält:
in einer ersten Alternative:
   - eine Lösung A enthaltend mindestens 0,2 M NaCl oder KCl, und SDS (Natriumdodecylsulfat) in einer Konzentration von 1 bis 2,5 Gew.-%,
   - eine Protease,
   - eine Lösung D enthaltend mindestens 2,5 M eines Salzes ausgewählt aus der Gruppe NaCl, KCl oder Na-Acetat,
   - eine Alkanollösung;
in einer zweiten Alternative:
   - eine Lösung A enthaltend mindestens 0,2 M NaCl oder KCl,
   - SDS (Natriumdodecylsulfat), wobei das Natriumdodecylsulfat in einer separaten Lösung mit einer Konzentration von 20 Gew.-% enthalten ist,
   - eine Protease,
   - eine Lösung D enthaltend mindestens 2,5 M eines Salzes ausgewählt aus der Gruppe NaCl, KCl oder Na-Acetat,
   - eine Alkanollösung;
in einer dritten Alternative:
   - eine Lösung A enthaltend mindestens 0,2 M NaCl oder KCl,
   - SDS (Natriumdodecylsulfat), wobei das Natriumdodecylsulfat als separate Festsubstanz enthalten ist,
   - eine Protease,
   - eine Lösung D enthaltend mindestens 2,5 M eines Salzes ausgewählt aus der Gruppe NaCl, KCl oder Na-Acetat,
   - eine Alkanollösung.

Dieser Kit erlaubt es dem Anwender, Nukleinsäuren, insbesondere genomische DNA aus den unterschiedlichsten organischen Materialien gemäß dem erfindungsgemäßen Verfahren zu isolieren und zu reinigen. Die Vorteile dieses Kits ergeben sich daraus, daß keine umweltgefährdenden Bestandteile, wie beispielsweise Phenol oder CTAB (Cetyltrimethylammoniumbromid) enthalten sind, die getrennt entsorgt werden müssen. Weiterhin sind in dem Kit auch keine teuren DNA-bindenden Säulchen enthalten, so daß bei der Verwendung des Kits für das erfindungsgemäße Verfahren auch kein nichtrecycelfähiger Plastikabfall anfällt.

In einer bevorzugten Ausführungsform enthält die Lösung A des Kits EDTA, 0,4 bis 1 M NaCl oder KCl, wobei der pH-Wert der Lösung auf 7,5 bis 8,5 eingestellt ist.

Erfindungsgemäß ist das Natriumdodecylsulfat (SDS) in der Lösung A in einer Konzentration von 1 bis 2,5 Gew-% oder als separate Festsubstanz oder in einer separaten Lösung mit einer Konzentration von 20 Gew.-% enthalten. Ist das SDS als separate Festsubstanz im Kit enthalten, so ist diese bereits abgewogen und der Anwender kann das SDS vor Beginn der DNA-Isolierung entweder zu der in einem definierten Volumen im Kit enthaltenen Lösung A zugeben, um eine Konzentration von 1 bis 2,5 Gew-% SDS zu erhalten oder durch Zugabe von Wasser oder TE-Puffer eine Lösung B (siehe Beispiele: Material) mit einer Konzentration von 10 bis 25 Gew.-% SDS herstellen.

Vorzugsweise ist die im Kit enthaltene Protease Proteinase K.

In einer besonders bevorzugten Ausführungsform beträgt die Konzentration des angegebenen Salzes in der Lösung D 5 M.

In einer weiteren besonders bevorzugten Ausführungsform des Kits ist zusätzlich RNAse als separate Festsubstanz enthalten. In der Regel wird die RNAse dem Kit als lyophilisiertes Pulver beigegeben. Der Anwender kann die bereits abgewogene RNAse vor Beginn der DNA-Isolierung entweder zu der in einem definierten Volumen im Kit enthaltenen Lösung A zugeben, um eine bestimmte Konzentration zu erhalten, oder eine separate RNAse-Lösung herstellen.

In einer weiteren besonders bevorzugten Ausführungsform enthält der Kit zusätzlich eine 70 bis 85 %ige Ethanol- oder Isopropanol-Lösung. Mit dieser Lösung kann die gefällte DNA behandelt werden, um sie von Salzen zu reinigen.

Weiterhin wird die technische Aufgabe gelöst durch Verwendung des erfindungsgemäßen Kits zur Isolierung und Reinigung von Nukleinsäuren, insbesondere von genomischer DNA aus einem organischen Material gemäß den Ansprüchen 1 bis 15. Die im Kit bereitgestellten Lösungen und Chemikalien sind so auf das erfindungsgemäße Verfahren abgestimmt, daß eine Isolierung genomischer DNA in kurzer Zeit aus den unterschiedlichsten organischen Materialien erfolgen kann.

In den nachfolgenden Beispielen soll das erfindungsgemäße Verfahren näher erläutert werden.

### Beispiele

In den folgenden Beispielen werden folgende Lösungen und Materialien verwendet:

### Material

| | |
|---|---|
| Lösung A | 0,4 M NaCl |
| | 10 mM Tris/HCl pH 8 |
| | 2 mM EDTA pH 8 |

- Lösung B: 20 % SDS (Natriumdodecylsulfat)
- Lösung C: 20 mg/ml Proteinase K (kühl aufbewahrt)

Lösung A : Lösung B : Lösung C im Verhältnis 1 : 0,1 : 0,02 wird kurz vor der Präparation fertig gemischt.
- Lösung D: 5 M NaCl-Lösung
- Isopropanol oder 100 % Ethanol pa zur Fällung
- 70 % Ethanol pa zur Reinigung der gefällten DNA
- steriles H₂O bidest oder 1 x TE-Puffer pH 8,0 (10 mM Tris, 1 mM EDTA)
- Wasserbad mit 65 °C, 37 °C
- Tischzentrifuge bis 14000 g
- sterile Einmalplastikartikel (2 ml Eppendorfcaps oder 50 ml Falcotubes)

### 1. Allgemeines Prinzip der Methode

Das die Nukleinsäuren enthaltende organische Material wird in einem Überschuß Lösung A enthaltend 0,4 M NaCl, 10 mM Tris/HCl pH 8 und 2 mM EDTA pH 8, aufgenommen. Dabei soll die Menge der Lösung in etwa dem doppelten Volumen des Substrats entsprechen. Ausgehend von einem Volumen der zuerst zugegebenen Lösung A wird 1/10 Volumen Lösung B (20 % SDS [Natriumdodecylsulfat]) und 2/100 Volumen Lösung C (Proteinase K, 20 mg/ml) zum Substrat gegeben und bei 65 °C für 15 bis 60 min inkubiert. Die weitere Isolation erfolgt bei Raumtemperatur. Bei kleinen Mengen Substrat, d.h. unter 0,5 g wird der komplette Überstand an Lysat weiterverarbeitet. Bei größeren Substratmengen werden nur 1 ml des flüssigen Lysats weiterverarbeitet. Falls das Lysat vom Substrat nicht getrennt abpipettiert werden kann, wird die Lösung für 5 min bei 2000 g in der Tischzentrifuge zentrifugiert. Zu dem Ansatz werden gemessen am Volumen des Ansatzes 0,75 Volumen der Lösung D (5 M NaCl) hinzugegeben und stark vermischt. Bei der anschließenden Zentrifugation für 10 min bei 14000 g werden Zellreste, Proteine und Polysaccharide abgetrennt. Der flüssige Überstand wird abgenommen und die darin befindliche DNA mit 1 Volumen Isopropanol bezogen auf das Volumen des abgezogenen Überstandes bei Raumtemperatur für 5 min gefällt. Nach der Zentrifugation der gefällten DNA für 10 min bei 14000 g wird der Überstand abpipettiert und die gefällte DNA mit 70 %igem Ethanol von Salzen gereinigt. Das Ethanol wird abpippetiert und das Pellet 15 min bei 37 °C getrocknet und dann in Wasser oder TE-Puffer (Tris/EDTA-Lösung) aufgenommen. Das ganze Verfahren dauert je nach Lysezeit etwa 60 bis 90 min.

### 2. Spezielle Präparationen

### a) Präparationen aus Pflanzen

Pflanzenteile, beispielsweise 0,1 g Blütenpollen werden in 200 µl, 2 Sojabohnen werden in 1 ml, 2 Gerstenkörner werden in 10 ml der Lösung A aufgenommen. Danach werden entsprechende Mengen Lösung B und C hinzugegeben und 60 min bei 65 °C lysiert. Eine Vorbehandlung stark ölhaltiger Pflanzen mit Hexan ist nicht erforderlich. Nach der Lyse wird unlösliches Material abgetrennt. Es kann bei sehr proteinreichen Pflanzen nach dem Salzschritt, d.h., nach Zugabe der 5 M NaCI-Lösung (Lösung D), eine Chloroformextraktion durchgeführt werden oder eine RNAse hinzugefügt werden, um RNA zu entfernen. Nach der Abtrennung des unlöslichen Materials erfolgt die Fällung der Nukleinsäuren. Anschließend wird die gefällte DNA ein oder zwei mal mit 70 %igem Ethanol gereinigt solange bis kein Salz mehr aus dem DNA-Pellet in Lösung geht. Kleine DNA-Pellets werden in 50 µl und große DNA-Pellets werden in bis zu 400 µl Wasser oder TE-Puffer aufgenommen.

### b) Präparation aus Gewebe und Fleisch

Gewebe (frisch, gefroren oder gegart) wird in ein ca. 1 cm² große Stücke geschnitten, entsprechen etwa 0,2 g und mit 400 µl der Lösung A, 40 µl der Lösung B und 8 µl Lösung C (20 mg/ml Proteinase K) aufgenommen und bei 65 °C für 60 min lysiert. Unlösliches Material wird abgetrennt und anschließend wird eine entsprechende Menge Lösung D hinzugegeben. Danach wird wieder unlösliches Material abgetrennt. Die Fällung der Nukleinsäure kann auch mit 2 Volumen 100 %igem Ethanol bezogen auf das Volumen des bisherigen Gesamtansatzes erfolgen. Vor der Fällung kann, falls erforderlich, die RNA für 15 min bei 37 °C mit einer RNAse verdaut werden (1/100 Vol. 100 µg/ml RNAse). Das DNA-Pellet wird schließlich in 100 µl Wasser oder TE-Puffer aufgenommen.

### c) Präparationen aus Lebensmitteln

Bei stark fetthaltigen Lebensmitteln kommt vor der Lyse eine Hexanbehandlung in Betracht, d.h., das Lebensmittel wird mit Hexan überschichtet und bei Raumtemperatur ca. 15 min geschüttelt. Das Hexan wird abpipettiert und dann erfolgt die Lyse, wie unter Beispiel 1 beschrieben. Feste Lebensmittel, zum Beispiel 100 mg Cornflakes werden in 400 µl Lösung A oder 2 g Müsli werden in 10 ml Lösung A aufgenommen. Eine Zerkleinerung durch Mörsern dieser festen Lebensmittel ist nicht notwendig, erhöht jedoch die Ausbeute an DNA. Flüssige Lebensmittel werden im Verhältnis 1 Volumen Substrat in 1,5 bis 2 Volumen Lösung A aufgenommen. Entsprechende Mengen Lösung B und C werden hinzugefügt. Zur Lyse des organischen Materials wird bei 65 °C für 60 min inkubiert und gegebenenfalls geschüttelt. Sollte kein flüssiger Überstand pippetierbar sein, so werden die festen Bestandteile für 5 min bei 2000 g zentrifugiert. Weiterhin kann bei zu starker Quellung des Lebensmittels auch mehr von der Lösung A nachgegeben werden. Möglicherweise bildet sich nach der Salzbehandlung mit Lösung D nach der Zentrifugation eine zusätzliche feste Schicht oberhalb der flüssigen Phase. Die flüssige Phase kann vorsichtig an dieser festen Schicht vorbei abgezogen werden. Danach erfolgt die Ethanolfällung. Das DNA-Pellet wird in der Regel in 100 µl Wasser oder TE-Puffer aufgenommen.

### d) Präparation von DNA aus grampositiven und gramnegativen Bakterien, Hefen und Schimmelpilzen

Die Mikroorganismen werden aus der Flüssigkeit gesammelt und je nach Ausgangsvolumen entweder durch Zentrifugation bei 3000 g oder durch Filtration mit einem Vakuumfiltrationsgerät und Filtern mit der Porengröße von mindestens 45 µm gesammelt. Von den Filtern werden die Mikroorganismen abgespült und durch eine weitere Zentrifugation konzentriert. Bei einer Mikroorganismenzahl, die einer Kolonie mittlerer Größe entspricht, werden die Mikroorganismen in 200 µl Lösung A aufgenommen. Bei einer größeren Ausgangsmenge werden 500 µl Lösung A eingesetzt. Entsprechende Mengen Lösung B und C werden hinzugefügt. Eine Inkubation bei 65 °C für 15 min reicht für die Lyse der Bakterien, für 60 min bei Hefen und Schimmelpilzen aus. Anschließend wird eine entsprechende Menge Lösung D hinzugefügt und unlösliches Material abgetrennt. Die DNA von Mikroorganismen wird mit 2 Volumen Ethanol bezogen auf das Volumen des Überstandes bei -25 °C für mindestens 15 min gefällt. Das Pellet wird mit 70 % Ethanol gereinigt, getrocknet und in 10 bis 100 µl Wasser aufgenommen.

### e) Präparation aus Zellkulturen

Die Zellkulturzellen werden vor der Präparation nicht trypsiniert und aus der Flasche herausgelöst, sondern bereits in der Flasche lysiert. Zunächst werden die Zellen mit 1 X PBS gespült. Dann werden die Lösungen A, B und C vorgemischt und in die Kulturflasche gegeben. Die ganze Flasche wird bei 65 °C für 60 min unter gelegentlichem Schwenken inkubiert. Das Lysat wird in ein 50 ml Zentrifugenröhrchen abgekippt und unlösliches Material abgetrennt. Anschließend wird die Salzbehandlung durchgeführt. Danach kann die Zentrifugation in den meisten Zentrifugen bei 5000 g, bevorzugt bei 8000 g für 10 min erfolgen. Nach der Isopropanolfällung wird die DNA in 200 bis 400 µl TE-Puffern gelöst und in ein kleineres Röhrchen überführt.

Das erfindungsgemäße Verfahren wurde zur Isolierung genomischer DNA aus den verschiedensten organischen Materialien durchgeführt. Aus der folgenden Auflistung ergeben sich die erfolgreichen DNA-lsolationen aus den genannten Materialien, wodurch die universelle Einsetzbarkeit des erfindungsgemäßen Verfahrens gezeigt wird:

**Bakterien** von Kulturplatten und aus Bier:
Pseudomonas sp., Escherichia coli, Lactobacillus sp., Megasphaera, Pectinatus, Allochromatium vinosum

**Hefen** von Kulturplatten, Flüssigkulturen, Trockensubstraten und aus Bier:
Saccharomyces sp., Schimmelpilze aus Tapeten, von Kulturplatten und von Obst.

**Tiere,** Frischfleisch (gefroren, gekocht od. gebraten; darunter beispielsweise Muskelfleisch, Haut, Fett, Leber, Niere, Schwanz, Ohr): der folgenden Spezies:
Schwein, Rind, Reh, Maus, Huhn, Pute, Fasan sowie Material aus Schnecke, Käfer, Fliege und Wespe

### Zellkultur

### Fibroblastenkulturzellen

**Pflanzen** (roh, gekocht, gefroren; Blätter, Stiele, Wurzeln, Blütenblätter, Pollen, Früchte) der folgenden Arten: Mais, Soja, Lupine, Raps, Zuckerrübe, Kartoffel, Tomate, Weizen, Gerste, Roggen, Hopfen, Tanne, Grünlilie, Birkenfeige, Zimmerpalme

### Lebensmittel

Sojaproteinisolate, Lecithin, Sojaöl kaltgepreßt, Margarine, Sojasauce, Gebäck, Sojaschnitzel, Sojawurst, Babynahrung, Sportlernahrung, Diätdrink, Backmischungen, vegetarischer Brotaufstrich, Maisstärke, Maltodextrin, Mehl, Cornflakes, Müsli, Müsliriegel, Popcorn, Tortillachips, Grieß, Waffeln, Polenta, Reispops, Milch, Sahne, Joghurt, Kakao, Schokolade, Pralinen, Tween 80, Honig, Salami, Cornedbeef und viele andere.

## Patentansprüche

1. Verfahren zur Isolierung und Reinigung von Nukleinsäuren, insbesondere von genomischer DNA, aus einem organischen Material, umfassend die Schritte:
a) Aufnehmen des Nukleinsäuren-enthaltenden organischen Materials in einer Lösung A enthaltend mindestens 0,2 M NaCl oder KCl,
b) Zugeben von SDS (Natriumdodecylsulfat)
c) Zugeben einer Protease
d) Inkubation für 5 bis 120 min bei 50 bis 75 °C
e) Zugeben einer Lösung D enthaltend ein Salz ausgewählt aus der Gruppe NaCl, KCl, Natriumacetat, wobei eine Endkonzentration des Salzes von mindestens 2,3 M erreicht wird.
f) Abtrennen des unlöslichen Materials
g) Fällen der Nukleinsäuren mit einem Alkanol.

2. Verfahren nach Anspruch 1, **dadurch gekennzeichnet, daß** nach den Schritten a) und/oder b) zusätzlich Inkubationen bei 50 bis 75 °C erfolgen.

3. Verfahren nach Anspruch 1 oder 2, **dadurch gekennzeichnet, daß** das Natriumdodecylsulfat und/oder eine Protease gemeinsam mit der Lösung A zugegeben werden.

4. Verfahren nach den Ansprüchen 1 bis 3, **dadurch gekennzeichnet, daß** als Protease Proteinase K verwendet wird.

5. Verfahren nach den Ansprüchen 1 bis 4, **dadurch gekennzeichnet, daß** zusätzlich RNAse zugegeben wird.

6. Verfahren nach den Ansprüchen 1 bis 5, **dadurch gekennzeichnet, daß** nach Schritt d) unlösliches Material abgetrennt wird.

7. Verfahren nach den Ansprüchen 1 bis 6, **dadurch gekennzeichnet, daß** die Inkubation in Schritt d) für 15 bis 60 min bei 65 °C erfolgt.

8. Verfahren nach den Ansprüchen 1 bis 7, **dadurch gekennzeichnet, daß** als Lösung A eine Lösung verwendet wird, enthaltend EDTA (Ethylendiamintetraessigsäure, Dinatriumsalz) und 0,4 bis 1 M NaCl oder KCl, wobei der pH-Wert der Lösung auf 7,5 bis 8,5 eingestellt ist.

9. Verfahren nach den Ansprüchen 1 bis 8, **dadurch gekennzeichnet, daß** die Endkonzentration des Natriumdodecylsulfats nach Zugabe 1,0 bis 2,5 Gew.-% beträgt.

10. Verfahren nach den Ansprüchen 1 bis 9, **dadurch gekennzeichnet, daß** als Lösung D eine 2,5 bis 6 M NaCl-Lösung verwendet wird.

11. Verfahren nach den Ansprüchen 1 bis 10, **dadurch gekennzeichnet, daß** als Alkanol Ethanol oder Isopropanol verwendet wird.

12. Verfahren nach den Ansprüchen 1 bis 11, **dadurch gekennzeichnet, daß** die Nukleinsäuren nach dem Fällen mit einer 70 bis 85 %igen Ethanol- oder Isopropanol-Lösung behandelt werden.

13. Verfahren nach den Ansprüchen 1 bis 12, **dadurch gekennzeichnet, daß** das organische Material ausgewählt ist aus der Gruppe prokaryotische Zellen, eukaryotische Zellen, Pflanzensamen, Pflanzenteile, Pflanzenöle, Nahrungsmittel, menschliche und tierische Gewebe, menschliche Ausscheidungen, Sperma, Speichel.

14. Kit zur Verwendung in dem Verfahren zur Isolierung und Reinigung von Nukleinsäuren gemäß den Ansprüchen 1 bis 13 enthaltend,
in einer ersten Alternative:
- eine Lösung A enthaltend mindestens 0,2 M NaCl oder KCl, und SDS (Natriumdodecylsulfat) in einer Konzentration von 1 bis 2,5 Gew.-%,
- eine Protease,
- eine Lösung D enthaltend mindestens 2,5 M eines Salzes ausgewählt aus der Gruppe NaCl, KCl oder Na-Acetat,
- eine Alkanollösung;
in einer zweiten Alternative:
- eine Lösung A enthaltend mindestens 0,2 M NaCl oder KCl,
- SDS (Natriumdodecylsulfat), wobei das Natriumdodecylsulfat in einer separaten Lösung mit einer Konzentration von 20 Gew.-% enthalten ist,
- eine Protease,
- eine Lösung D enthaltend mindestens 2,5 M eines Salzes ausgewählt aus der Gruppe NaCl, KCl oder Na-Acetat,
- eine Alkanollösung;
in einer dritten Alternative:
- eine Lösung A enthaltend mindestens 0,2 M NaCl oder KCl,
- SDS (Natriumdodecylsulfat), wobei das Natriumdodecylsulfat als separate Festsubstanz enthalten ist,
- eine Protease,
- eine Lösung D enthaltend mindestens 2,5 M eines Salzes ausgewählt aus der Gruppe NaCl, KCl oder Na-Acetat,
- eine Alkanollösung.

15. Kit nach Anspruch 14, **dadurch gekennzeichnet, daß** die Lösung A EDTA (Ethylendiamintetraessigsäure, Dinatriumsalz) und 0,4 bis 1 M NaCl oder KCl enthält, und der pH-Wert der Lösung 7,5 bis 8,5 beträgt.

16. Kit nach den Ansprüchen 14 oder 15, **dadurch gekennzeichnet, daß** die Protease Proteinase K ist.

17. Kit nach den Ansprüchen 14 bis 16, **dadurch gekennzeichnet, daß** die Konzentration des angegebenen Salzes in der Lösung D 5 M ist.

18. Kit nach den Ansprüchen 14 bis 17, **dadurch gekennzeichnet, daß** zusätzlich eine 70 bis 85 %ige Ethanol- oder Isopropanol-Lösung enthalten ist.

19. Kit nach den Ansprüchen 14 bis 18, **dadurch gekennzeichnet, daß** zusätzlich RNAse enthalten ist.

20. Verwendung des Kits nach den Ansprüchen 14 bis 19 zur Isolierung und Reinigung von Nukleinsäuren, insbesondere von genomischer DNA, aus einem organischen Material gemäß den Ansprüchen 1 bis 13.

## Claims

1. A process for isolating and purifying nucleic acids, in particular genomic DNA, from an organic material, comprising the following steps:
a) taking up the nucleic acid-containing organic material in a solution A containing at least 0.2 M NaCl or KCl;
b) adding SDS (sodium dodecyl sulfate);
c) adding a protease;
d) incubating for from 5 to 120 min at from 50 to 75°C;
e) adding a solution D containing a salt selected from the group NaCl, KCl and sodium acetate, with the salt reaching a final concentration of at least 2.3 M;
f) Separating off the insoluble material;
g) precipitating the nucleic acids with an alkanol.

2. The process according to claim 1, wherein incubations at from 50 to 75°C additionally take place after steps a) and/or b).

3. The process according to claim 1 or 2, wherein the sodium dodecyl sulfate and/or a protease is/are added together with the solution A.

4. The process according to claims 1 to 3, wherein proteinase K is used as the protease.

5. The process according to claims 1 to 4, wherein RNAse is additionally added.

6. The process according to claims 1 to 5, wherein insoluble material is separated off after step d).

7. The process according to claims 1 to 6, wherein the incubation in step d) takes place at 65°C for from 15 to 60 min.

8. The process according to claims 1 to 7, wherein a solution containing EDTA (ethylenediamine-tetraacetic acid, disodium salt) and from 0.4 to 1 M NaCl or KCl, with the pH of the solution being adjusted to from 7.5 to 8.5, is used as solution A.

9. The process according to claims 1 to 8, wherein the final concentration of the sodium dodecyl sulfate after addition is from 1.0 to 2.5% by weight.

10. The process according to claims 1 to 9, wherein a NaCl solution of from 2.5 to 6 M is used as solution D.

11. The process according to claims 1 to 10, wherein ethanol or isopropanol is used as the alkanol.

12. The process according to claims 1 to 11, wherein, after having been precipitated, the nucleic acids are treated with an ethanol or isopropanol solution which is from 70 to 85% in concentration.

13. The process according to claims 1 to 12, wherein the organic material is selected from the group prokaryotic cells, eukaryotic cells, plant seeds, plant parts, plant oils, foodstuffs, human and animal tissues, human excreta, sperm and saliva.

14. A kit for use in the process for isolating and purifying nucleic acids as claimed in claims 1 to 13, comprising
in a first alternative:
- a solution A containing at least 0.2 M NaCl or KCl and SDS (sodium dodecyl sulfate) at a concentration of from 1 to 2.5% by weight,
- a protease,
- a solution D containing at least 2.5 M of a salt selected from the group NaCl, KCl and Na-acetate,
- an alkanol solution;
in a second alternative:
- a solution A containing at least 0.2 M NaCl or KCl;
- SDS (sodium dodecyl sulfate), wherein the sodium dodecyl sulfate is provided in a separate solution having a concentration of 20% by weight,
- a protease,
- a solution D containing at least 2.5 M of a salt selected from the group NaCl, KCl and Na-acetate,
- an alkanol solution;
in a third alternative:
- a solution A containing at least 0.2 M NaCl or KCl;
- SDS (sodium dodecyl sulfate), wherein the sodium dodecyl sulfate is provided separately as solid substance;
- a protease,
- a solution D containing at least 2.5 M of a salt selected from the group NaCl, KCl and Na-acetate,
- an alkanol solution.

15. The kit according to claim 14, wherein the solution A contains EDTA (ethylenediamine-tetraacetic acid, disodium salt) and from 0.4 to 1 M NaCl or KCl, and the pH of the solution is from 7.5 to 8.5.

16. The kit according to claim 14 or 15, wherein the protease is proteinase K.

17. The kit according to claims 14 to 16, wherein the concentration of the given salt in solution D is 5 M.

18. The kit according to claims 14 to 17, wherein an ethanol or isopropanol solution of from 70 to 85% in concentration is additionally present.

19. The kit according to claims 14 to 18, wherein RNAse is additionally present.

20. The use of the kit of in claims 14 to 19 for isolating and purifying nucleic acids, in particular genomic DNA, from an organic material as claimed in claims 1 to 13.

## Revendications

1. Procédé d'isolation et de purification d'acides nucléiques, en particulier d'ADN génomique à partir d'un matériel organique, comprenant les étapes suivantes :
a) absorption du matériel organique contenant les acides nucléiques dans une solution A contenant au moins 0,2 M de NaCl ou de KCl,
b) addition de SDS (dodécylsulfate de sodium),
c) addition d'une protéase,
d) incubation pendant 5 à 120 minutes à 50 - 75°C
e) addition d'une solution D contenant un sel, choisi dans le groupe constitué de NaCl, KCl, acétate de sodium, avec une concentration finale du sel d'au moins 2,3 M atteinte,
f) séparation du matériel insoluble,
g) précipitation des acides nucléiques avec un alcanol.

2. Procédé selon la revendication 1,
**caractérisé en ce qu'**
après les étapes a) et/ou b), on procède en complément à des incubations à 50 - 75°C.

3. Procédé selon la revendication 1 ou 2,
**caractérisé en ce qu'**
on ajoute le dodécylsulfate de sodium ou une protéase conjointement à la solution A.

4. Procédé selon les revendications 1 à 3,
**caractérisé en ce qu'**
on utilise la protéinase K en tant que protéase.

5. Procédé selon les revendications 1 à 4,
**caractérisé en ce qu'**
on ajoute en plus une RNase.

6. Procédé selon les revendications 1 à 5,
**caractérisé en ce qu'**
après l'étape d), on sépare le matériel insoluble.

7. Procédé selon les revendications 1 à 6,
**caractérisé en ce que**
l'incubation à l'étape d) est réalisée pendant 15 à 60 min à 65°C.

8. Procédé selon les revendications 1 à 7,
**caractérisé en ce qu'**
on utilise comme solution A une solution contenant de l'EDTA (acide éthylènediamine tétracétique, sel disodique) et 0,4 à 1 M de NaCl ou de KCl, le pH de la solution étant ajusté sur 7,5-8,5.

9. Procédé selon les revendications 1 à 8,
**caractérisé en ce que**
la concentration finale du dodécylsulfate de sodium est de 1,0 à 2,5 % en poids après l'addition.

10. Procédé selon les revendications 1 à 9,
**caractérisé en ce qu'**
on utilise comme solution D une solution de NaCl à 2,5 - 6 M.

11. Procédé selon les revendications 1 à 10,
**caractérisé en ce qu'**
on utilise comme alcanol de l'éthanol ou de l'isopropanol.

12. Procédé selon les revendications 1 à 11,
**caractérisé en ce qu'**
après la précipitation, on traite les acides nucléiques avec une solution à 70 - 85 % d'éthanol ou d'isopropanol.

13. Procédé selon les revendications 1 à 12,
**caractérisé en ce qu'**
on choisit le matériel organique dans le groupe constitué de cellules procaryotiques, de cellules eucaryotiques, de graines de plantes, de parties de plantes, d'huiles végétales, d'aliments, de tissus humains ou d'origine animale, d'excrétions humaines, de sperme et de salive.

14. Kit à utiliser dans le procédé d'isolation et de purification d'acides nucléiques selon les revendications 1 à 13, contenant,
dans une première variante :
- une solution A contenant au moins 0,2 M de NaCl ou de KCl et du SDS (dodécylsulfate de sodium) dans une concentration de 1 à 2,5 % en poids,
- une protéase,
- une solution D contenant au moins 2,5 M d'un sel choisi dans le groupe constitué de NaCl, KCl ou acétate de Na,
- une solution d'alcanol ;
dans une deuxième variante :
- une solution A contenant au moins 0,2 M de NaCl ou de KCl,
- du SDS (dodécylsulfate de sodium), le dodécylsulfate de sodium se trouvant dans une solution séparée à une concentration de 20 % en poids,
- une protéase,
- une solution D contenant au moins 2,5 M d'un sel choisi dans le groupe constitué de NaCl, KCl ou acétate de Na,
- une solution d'alcanol ;
dans une troisième variante :
- une solution A contenant au moins 0,2 M de NaCl ou de KCl,
- du SDS (dodécylsulfate de sodium), le dodécylsulfate de sodium étant présent en tant que substance solide séparée,
- une protéase,
- une solution D contenant au moins 2,5 M d'un sel choisi dans le groupe constitué de NaCl, KCl ou acétate de Na,
- une solution d'alcanol.

15. Kit selon la revendication 14,
**caractérisé en ce que**
la solution A contient de l'EDTA (acide éthylènediamine tétracétique sel disodique) et 0,4 à 1 M de NaCl ou de KCl, et le pH de la solution est de 7,5 à 8,5.

16. Kit selon les revendications 14 ou 15,
**caractérisé en ce que**
la protéase est la protéinase K.

17. Kit selon les revendications 14 ou 16,
**caractérisé en ce que**
la concentration du sel indiqué dans la solution D est de 5 M.

18. Kit selon les revendications 14 ou 17,
**caractérisé en ce qu'**
une solution d'éthanol ou d'isopropanol à 70 - 85 % est présente en complément.

19. Kit selon les revendications 14 à 18,
**caractérisé en ce qu'**
il y a, en complément, de la RNase.

20. Utilisation du kit selon les revendications 14 à 19, pour isoler et purifier des acides nucléiques, en particulier de l'ADN génomique, à partir d'un matériel organique selon les revendications 1 à 13.
